# EUROPEAN PATENT SPECIFICATION

(11) **EP 1 579 870 B1**
(45) Date of publication and mention of the grant of the patent: **10.06.2009**
(21) Application number: 03786265.3
(22) Date of filing: 24.12.2003
(51) Int. Cl.: A61K 39/00, A61K 48/00, A61P 35/00

(54) **VACCINES COMPRISING POLYNUCLEOTIDES**
POLYNUKLEOTIDHALTIGE IMPFSTOFFE
VACCINS COMPRENANT DES POLYNUCLEOTIDES

(30) Priority: 24.12.2002 JP 2002372103
(43) Date of publication of application: 28.09.2005
(73) Proprietor: ImmunoFrontier, Inc., Tokyo 103-0025 (JP)
(72) Inventor: SHIKU, Hiroshi, Tsu-shi, Mie 514-0061 (JP)
(74) Representative: Bentham, Andrew
(86) International application number: PCT/JP2003/016548
(87) International publication number: WO 2004/058299

(56) References cited:
- WO-A-01/24764
- WO-A-03/000894
- WO-A2-01/83750
- JP-A- 2000 302 692
- NISHIKAWA H ET AL: "Role of SEREX-defined immunogenic wild-type cellular molecules in the development of tumor-specific immunity" PROCEEDINGS OF THE NATIONAL ACADEMY OF SCIENCES OF USA, NATIONAL ACADEMY OF SCIENCE, WASHINGTON, DC, US, vol. 98, no. 25, 4 December 2001 (2001-12-04), pages 14571-14576, XP002960469 ISSN: 0027-8424
- ELKE JÄGER ET AL: "Induction of primary NY-ESO-1 immunity: CD8+ T lymphocyte and antibody responses in peptide-vaccinated patients with NY-ESO-1+ cancers" PROCEEDINGS OF THE NATIONAL ACADEMY OF SCIENCES OF USA, NATIONAL ACADEMY OF SCIENCE, WASHINGTON, DC, US, vol. 97, no. 22, 24 October 2000 (2000-10-24), pages 12198-12203, XP002245753 ISSN: 0027-8424
- CHEN Y-T ET AL: "A TESTICULAR ANTIGEN ABERRANTLY EXPRESSED IN HUMAN CANCERS DETECTED BY AUTOLOGOUS ANTIBODY SCREENING" PROCEEDINGS OF THE NATIONAL ACADEMY OF SCIENCES OF USA, NATIONAL ACADEMY OF SCIENCE, WASHINGTON, DC, US, vol. 94, 1997, pages 1914-1918, XP000960590 ISSN: 0027-8424
- JAEGER E ET AL: "Vaccines in breast cancer" BREAST, vol. 10, no. Supplement 3, August 2001 (2001-08), pages 158-160, XP009067502 ISSN: 0960-9776
- NISHIKAWA H, ET AL: 'ROLE OF SEREX-DEFINED IMMUNOGENIC WILD-TYPE CELLULAR MOLECULES IN THE DEVELOPMENT OF TUMOR-SPECIFIC IMMUNITY' PROCEEDINGS OF THE NATIONAL ACADEMY OF SCIENCES OF UNITED STATES OF AMERICA vol. 98, no. 25, 2001, pages 14571 - 14576, XP002960469

## Description

### Technical Field

The present invention relates to vaccines for developing tumor-specific immunity *in vivo.*

### Background Art

For more than a decade, cancer has been the leading cause of death in Japan. Immunotherapy is expected to be a fourth method of cancer treatment, along with surgery, radiotherapy and chemotherapy. Immunotherapy is a treatment method utilizing the *in vivo* immune response system. An immune response is elicited and controlled by interactions between B lymphocytes, T lymphocytes, antibodies, and antigen presenting cells (APC). First, an exogenous antigen is processed by an APC, bound to a major histocompatibility complex (MHC) class I or class II molecule, and presented to helper T cells. Helper T cell recognition of this MHC-bound exogenous antigen leads to T cell activation and cytokine secretion. The secreted cytokines help differentiate antigen-stimulated B cells into antibody-forming cells and also promote the differentiation of killer T cells. Cells expressing the antigens are eliminated by secreted antibodies and activated killer T cells, and thus cellular and humoral responses progress to eliminate exogenous antigens.

Elimination of antigen-expressing cells by T cells can classified into three main groups: 1) humoral immunity (activated helper T cells stimulate proliferation/differentiation of specific B cell clones to produce antibodies that function in the recognition and elimination of antigens), 2) specific cellular immunity (activated helper T cells induce cytotoxic T cells (CTL) that respond in an antigen-specific manner and act directly on the target), and 3) non-specific cellular immunity (activated helper T cells induce non-specific natural killer cells, activated macrophages, and such, and these cells function to eliminate antigens). As described above, T cells play a central role in recognizing target antigens to elicit an immune response.

Regarding tumor rejection, appropriate immunization using congenital or self-derived tumor cells or their fractions has been known to induce antitumor immune responses in host cells (L. Gross, Cancer Res. 3: 326-333 (1943); E.J. Foley, Cancer Res. 13: 835-837 (1953); R.T. Prehn and J.M. Maln, J. Natl. Cancer Inst. 18: 769-778 (1957); G. Klein et al., Cancer Res. 20: 1561-1572 (1980); L. Old et al., Ann. N.Y. Acad. Sci. 101: 80-106 (1962); A. Globerson and M. Feldman, J. Natl. Cancer Inst. 32: 1229-1243 (1964)). The role of CD8+ and CD4+ T cells in these antitumor immune systems has been of enormous interest (R.J. North, Adv. Immunol. 35: 89-155 (1984); P.D. Greenberg, Adv. Immunol. 49: 281-355 (1991); D.M. Pardoll and S.L. Topalian, Curr. Opin. Immunol. 10: 588-594 (1998)). CD8+ T cells derived from specifically immunized mice are reported to be capable of destroying tumor target cells *in vitro* (H. Wagner et al., Adv. Cancer Res. 31: 77-124 (1980)). It has also been reported that adoptive transfer of CD8+ T cells from an immunized donor confers resistance to tumor transplants to susceptible mice (R.J. North, Adv. Immunol. 35: 89-155 (1984); P.D. Greenberg, Adv. Immunol. 49: 281-355 (1991); C. J.M. Melief, Adv. Cancer Res. 58: 143-175 (1992)). In addition, anti-CD8+ antibodies are known to abolish resistance to tumor transplantation in preimmunized mice (E. Nakayama and A. Uenaka, J. Exp. Med. 161: 345-355 (1985); X.G Gu et al., Cancer Res., 58: 3385-3390 (1998); Y. Noguchi et al., Proc. Natl. Acad. Sci. USA 92: 2219-2223 (1994)). Over the past decade, MHC class I binding peptides derived from the tumor cells of mice and humans and recognized by CD8+ T cells have been discovered (T. Boon et al., Annu. Rev. Immunol. 12: 337-368 (1994); S.A. Rosenberg, Immunity 10: 281-287 (1999)).

There are two forms of tumor antigen (i.e., target molecules on tumor cells): tumor peptides presented by MHC class I molecules and targeted by CD8+ CTL, which plays a major part in cellular immunity (tumor rejection antigens); and molecules called tumor-associated antigens, which are targeted by humoral immunity (antibodies) and expressed on tumor cell membranes. Following the genetic level identification of human tumor antigens recognized by T lymphocytes, various human tumor rejection antigens have been discovered. In melanomas, a clear antitumor effect has been observed due to vaccination therapy, which is a specific immunotherapy using tumor rejection antigens. Furthermore, attempts are being made to enhance the immunotherapeutic effect by the combined use of cytokines, and to apply dendritic cells pulsed with antigenic peptides, or dendritic cells to which an antigen gene has been introduced. Recently, DNA vaccines are also being tested.

A number of approaches have been attempted to augment the helper action of CD4+ T cells (D. Pardoll and S.L. Topalian, Curr. Opin. Immunol. 10: 588-594 (1998); R.F. Wang, Trends Immunol. 5: 269-276 (2001)). Earlier methods can be divided into three categories. The first method is modification of the immunizing antigens themselves by, for example, haptenizing an antigen (Y. Mizushima et al., J. Natl. Cancer Inst. 74: 1269-1273 (1985)) or directly linking a heterologous immunogenic peptide to an antigen (R.W. Chesnut et al., Vaccine Design, eds. M.F. Powell and M.J. Newman (Plenum, New York) 847-874 (1995); J. Rice et al., J. Immunol. 167: 1558-1565 (2001)). The second method is co-immunization with tumor antigens and molecules having a strong helper determinant (R. Romieu et al., J. Immunol. 161: 5133-5137 (1998); N. Casares et al., Eur. J. Immunol. 31: 1780-1789 (2001)), such as viral vectors encoding tumor antigens (M. Wang et al., J. Immunol. 154: 4685-4692 (1995)). The last method is based on the discovery of molecular signals such as CD40 ligands (J.P. Ridge et al., Nature (London) 393: 474-478 (1998); S.R.M. Bennett et al., Nature (London) 393: 478-480 (1998); S.P. Schoenberg et al., Nature (London) 393: 480-483 (1998)) and other stimulatory or co-stimulatory signals regulating the helper function of CD4+ T cells and the interaction of APC with CD4+ T cells (A. Porgador et al., J. Exp. Med. 188: 1075-1082 (1998)). The discovery of such signals is thought to provide methods for augmenting CD8+ T cell response.

Conventionally, antibodies have not been thought to play a large part in antitumor effector functions. However, it is now clear that tumor antigens elicit a strong and complete immune response involving both cellular and humoral immunity (for example, Y-T. Chen et al., Proc. Natl. Acad. Sci. USA 94: 1914-1918 (1997); E. Jager et al., J. Exp. Med. 187: 625-630 (2000); E. Jager et al., Proc. Natl. Acad. Sci. USA 97: 12198-12203 (2000)). Recently, M. Pfreundschuh and his colleagues developed a method called SEREX (Y.-T. Chen et al., Proc. Natl. Acad. Sci. USA 94: 1914-1918 (1997)) to identify tumor-associated antigens that may serve as vaccines against tumors. In this method, human sera are used to screen human tumor cDNA expression libraries. More than 1800 kinds of genes identified by SEREX are registered on the Internet as the SEREX database (www.licr.org/SEREX.html).

However, many problems are left unsolved, such as what kind of adjuvant or APC to use to effectively induce tumor-specific immunity using the identified antigenic peptides/DNAs to completely treat tumors; or how to deal with the immune system evasion of tumors.

Helper T cells are often reported to be necessary for quantitative/qualitative amplification of CTL. However, little is known about the characteristics of antigen molecules recognized by these T cells and the functional impact of T cells on antitumor immune responses (P.D. Greenberg, Adv. Immunol. 49: 281-355 (1991); D.M. Pardoll and S.L. Topalian, Curr. Opin. Immunol. 10: 588-594 (1998); S.R. Bennett et al., J. Exp. Med. 186: 65-70 (1997); R.F. Wang, Trends Immunol. 5: 269-276 (2001); C. Fayolle et al., J. Immunol. 147: 4069-4073 (1991); M. Shiral et al., J. Immunol. 152: 1549-1556 (1994); K. Hung et al., J. Exp. Med. 188: 2357-2368 (1998); F. Ossendorp et al., J. Exp. Med. 187: 693-702 (1998); Y. Shen and S. Fujimoto, Cancer Res. 56: 5005-5011 (1996); T. Nishimura et al., J. Exp. Med. 190: 617-627 (1999); D.R. Surman et al., J. Immunol. 164: 562-565 (2000); A. Franco et al., Nat. Immunol. 1: 145-150 (2000); C.N. Baxevanis et al., J. Immunol. 164: 3902-3912 (2000); F. Fallarino et al., J. Immunol. 165: 5495-5501 (2000); A.L. Marzo et al., Cancer Res. 59: 1071-3390 (1999); A.L. Marzo et al., J. Immunol. 165: 6047-6055 (2000)). The current hypothesis for serial intercellular interaction amongst helper T cells, CTL and APC points to the possibility that helper T cells related to antitumor immune response can recognize a diverse and wide range of antigens (J.P. Ridge et al., Nature 393: 474-478 (1998); S.R.M. Bennett et al., Nature 393: 478-480 (1998); S.P. Schoenberger et al., Nature 393: 480-483 (1998); Z. Lu et al., J. Exp. Med. 191: 541-550 (2000)).

Analyses of humoral immune responses in human and murine tumors are greatly advancing due to the above-described SEREX method (Y.-T. Chen et al., Proc. Natl. Acad. Sci. USA 94: 1914-1918 (1997); E. Jager et al., J. Exp. Med. 187: 625-630 (2000); E. Jager et al., Proc. Natl. Acad. Sci. USA 97: 12198-12203 (2000); U. Sahin et al., Proc. Natl. Acad. Sci. USA 92: 11810-11813 (1995); L.J. Old and Y.-T. Chen, J. Exp. Med. 187: 1163-1167 (1998); Y.-T. Chen, "Principle and Practice of the Biologic Therapy of Cancer", ed. S.A. Rosenberg (Lippincott Williams & Wilkins, Philadelphia) 557-570 (2000); T. Ono et al., Int. J. Cancer 88: 845-851 (2000)). It is reported that when many of the genes identified by SEREX are completely sequenced, their sequence is the same as the wild-type sequence. In other words, they do not comprise amino acid substitutions or such (L.J. Old and Y.-T. Chen, J. Exp. Med. 187: 1163-1167 (1998); Y.-T. Chen, "Principle and Practice of the Biologic Therapy of Cancer", ed. S.A. Rosenberg (Lippincott Williams & Wilkins, Philadelphia) 557-570 (2000)). Therefore, the immunogenicity of these molecules is not the result of mutations. Furthermore, although some SEREX antigens show tumor-restricted expression in normal tissues (e.g., cancer/testis antigens, melanocyte differentiation antigens, etc.), most of the SEREX-identified antigens are ubiquitously expressed. However, the serum samples of patients with associated or non-associated cancer show more antibodies with a high titer against these wild-type molecules than normal healthy subjects. At present, the immunogenic stimulus for eliciting humoral immunity is thought to be the enhanced expression of these tumor products. Since all of these molecules are detected by IgG class antibodies, it is possible that CD4+ helper T cells recognize these wild-type molecules. In view of the above, the present inventors examined and discovered that wild-type immunogenic molecules of tumor cells can amplify tumor-specific CD8+ CTL via CD4+ helper T cell activation. Namely, they discovered the involvement of such molecules in antitumor immune response (H. Nishikawa et al., Pro. Natl. Acad. Sci. U.S.A. 98(25):14571-14576 (2001)).

With regards to DNA vaccines, intramuscular administration of naked DNA has been proven to induce both humoral and cellular immune responses. Though the precise mechanism by which DNA vaccines induce an immune response is currently unknown (see Pardoll et al., Immunity 3: 165-169 (1995)), their effectiveness is shown by the induction of humoral and cellular immunities. These results indicate that naked DNA is expressed after administering a DNA vaccine, and peptide products of the naked DNA are presented as antigens with both the MHC class I and class II proteins.

Exogenous peptides bound to MHC class I and/or II molecules and derived from viruses, bacteria, and the like are recognized as antigens by T cell receptors on CTL. This promotes reactions such as the production of various lymphokines and proliferation of cells, killing cells infected with the virus, bacteria, or such from which the exogenous peptide was derived. Irrespective of the location or function in the pathogen, these antigenic peptides are fragments incorporated and processed by APC or other cells. To artificially generate CTL responses, replication vectors that produce protein antigens in cells can be used (J.R. Bennink and J.W. Yewdell, Curr. Top. Microbiol. Immunol. 163: 153 (1990); C.K. Stover et al., Nature 351: 456 (1991); A. Aldovini and R.A. Young, Nature 351: 479 (1991); R. Schfer et al., J. Immunol. 149: 53 (1992); C.S. Hahn et al., Proc. Natl. Acad. Sci. USA 89: 2679 (1992)); as well as methods for introducing peptides to the cytosol (F.R. Carbone and M.J. Bevan, J. Exp. Med. 169: 603 (1989); K. Deres et al., Nature 342: 561 (1989); H. Takahashi et al., Nature 344: 873 (1990); D.S. Collins et al., J. Immunol. 148: 3336 (1992); M.J. Newman et al., J. Immunol. 148: 2357 (1992)).

Furthermore, a method for using naked polynucleotides as a vaccine to inoculate a vertebrate has been examined (WO90/11092 (October 4, 1990)). Expression of DNA precipitated with calcium chloride and administered into the abdominal cavity, veins, or muscles is possible (N. Benvenisty and L. Reshef, Proc. Natl. Acad. Sci. USA 83: 9551-9555 (1986)). In mice, a DNA expression vector was shown to be incorporated into myocytes and expressed in cells upon intramuscular injection (J.A. Wolff et al., Science 247: 1465 (1990); G. Ascadi et al., Nature 352: 815 (1991)). In this case, the vector was maintained as an episome and did not replicate. However, persistent expression of the vector was observed following injection into the skeletal muscle of rats, fish, and primates, as well as the cardiac muscle of rats (H. Lin et al., Circulation 82: 2217 (1990); R.N. Kitsis et al., Proc. Natl. Acad. Sci. USA 88: 4138 (1991); E. Hansen et al., FEBS Lett. 290: 73 (1991); S. Jiao et al., Hum. Gene Therapy 3: 21 (1992); J.A. Wolff et al., Human Mol. Genet. 1: 363 (1992)). It was further reported that B7 on the surface of APC and MHC presentation of epitopes play comparable roles in the activation of CTL during tumor elimination (Edington, Biotechnology 11: 1117-1119 (1993)). When an MHC molecule on the surface of APC presents an epitope to a T cell receptor, B7 expressed on the surface of the same APC binds to CTLA-4 or CD28 and functions as the second signal. As a result, CD4+ helper T cells, which emit signals to increase APC-destroying CD8+ T cells, rapidly proliferate.

To immunize with DNAs, the DNAs do not necessarily have to be administered intramuscularly. For example, Tang *et al.* demonstrates that mice produce anti-bovine growth hormone (BGH) antibodies following administration to the skin of gold particles coated with DNA coding for BGH (Tang et al., Nature 356: 152-154 (1992)). In addition to skin, it is reported that a jet injector can be used to transfect DNA to living animal tissues, such as muscular, adipose, mammary gland tissues and such (Furth et al., Analytical Biochemistry 205: 365-368 (1992)). Various methods for introducing nucleic acids have also been published as reviews (T. Friedman, Science 244: 1275-1281 (1989)). WO93/17706 describes a method for inoculating an animal with a vaccine against viruses, by coating a carrier particle with a gene construct, and administering the coated particle to a cell of the animal. Further, DNA immunization against herpes virus has been reported (Cox et al., J. Virol. 67: 5664-5667 (1993)). In addition, DNA vaccines and their production and/or administration methods are also described in U.S. Patent No. 4,945,050, U.S. Patent No. 5,036,006, U.S. Patent No. 5,589,466, WO94/16737, and such.

### Disclosure of the Invention

An objective of the present invention is to provide compositions for effective immunotherapy against tumors. Additional objectives include providing compositions for permanent treatments for early stage cancers, compositions for suppressing postoperative recurrence or metastasis of cancers, and compositions for treating patients whose tumor has been detected but is inoperable, and for whom radiation and chemical treatments were ineffective.

The present invention provides:
- a composition comprising one or more expression vectors encoding an antigen recognized by CD4+ helper T cells, and a tumor-specific or tumor-associated antigen; wherein the antigen recognized by CD4+ helper T cells is NY-ESO-1, and the tumour-specific or tumour-associated antigen is HER-2/neu;
- for the use of such a composition in the manufacture of a medicament for inducing tumor-specific immunity in a mammal; and
- such compositions for inducing tumor specific immunity in an animal.

The invention relates to polynucleotide vaccines comprising one or more expression vectors that encode an antigen recognized by CD4+ helper T cells, and a tumor-specific or tumor-associated antigen. The antigen recognized by CD4+ helper T cells is NY-ESO-1. The tumour-specific or tumour-associated antigen is HER-2/neu. To reliably achieve activation of CD4+ helper T cells for developing tumor immunity, an expression vector encoding interferon gamma is preferably administered at the same time. The tumor-specific or tumor-associated antigen may show high rates of expression in one or more cancer types, and comprising an antigenic site that exhibits suitable compatibility with, and thus binds to, major histocompatibility complex (MHC) class I molecules of mammals including humans. Furthermore, so that they can be expressed and/or presented on the same cell, the expression vectors encoding the antigen recognized by CD4+ helper T cells and the tumor-specific or tumor-associated antigen, as well as expression vectors encoding interferon gamma are preferably immobilized on the same carrier particle or the like and administered to a cell; however, this invention is not restricted to this.

The present invention relates to vaccines for developing tumor-specific immunity, and is based on the finding that tumor-specific immunity can be induced by using, as a polynucleotide vaccine, expression vectors encoding an antigen recognized by CD4+ helper T cells, and a tumor-specific or tumor-associated antigen, and preferably an expression vector that encodes interferon gamma. Thus; the present invention provides a composition comprising one or more expression vectors encoding an antigen recognized by CD4+ helper T cells, and a tumor-specific or tumor-associated antigen, wherein the antigen recognized by CD4+ helper T cells is NY-ESO-1 and the tumour-specific or tumour-associated antigen is HER-2/neu. More preferably, the composition further comprises an expression vector encoding interferon gamma. The present inventors have shown that simultaneous presentation of a SEREX antigen with a tumor-specific or tumor-associated antigen strongly induces tumor-specific immunity (H. Nishikawa et al., Pro. Nat. Acad. Sci. USA. 98(25):14571 (2001)). The inventors have also found that independent expression of only a SEREX antigen suppresses CD4+ helper T cell activity, thus suppressing tumor-specific immunity, whereas co-expression with interferon gamma blocks such suppression (Japanese Patent Application No. 2002-254967; H. Nishikawa et al., Proc. Nat. Acad. Sci. USA. 100(19):10902 (2003)).

Thus, expression vectors encoding an antigen recognized by CD4+ helper T cells and a tumor-specific or tumor-associated antigen, and, more preferably an additional expression vector encoding interferon gamma, are immobilized onto the same carrier particles and administered to cells so as to be expressed and presented on the same cell. However, this invention is not limited to this.

As described herein, an "antigen recognized by CD4+ helper T cells" refers to a polypeptide that comprises a portion that serves as an epitope of a CD4+ glycoprotein expressed on mature helper T cells or precursor cells thereof. When a T cell receptor recognizes an antigen presented by an MHC class II, CD4 binds to the β2 domain of the MHC class II and thus enhances the ability of the T cell to recognize antigens. CD4 further transmits signals into the cell, and promotes proliferation and secretion of cytokines. The antigen recognised by CD4 + helper T cells is NY-ESO-1.

Herein, a "tumor-specific or tumor-associated antigen" in the present invention refers to an antigen that is associated with tumors or expressed specifically in specific tumor cells. The tumour-specific or tumour-associated antigen used in the invention is HER-2/neu.

Antigens expressed only in cancers and testis include MAGE, BAGE, GAGE, SAGE, NY-ESO-1, etc. Cancer-specific mutant antigens, which are derived from mutant gene products produced during carcinogenesis, include CDK4, MUM-1, CASP-8, ras, bcr-abl, etc. Tissue-specific antigens, which are locally expressed in specific tissues, include MART-1, TRP, tyrosinase, gp100, PSA, proteinase 3, etc. Protein antigens expressed in higher levels in cancers include HER2/neu, CEA, SART1, etc. Viral antigens include EBV, HPV, HTLV-1, etc. Herein, a tumor-specific, tumor-associated antigen may be a full-length protein or a fragment of such with immunogenicity.

Tumor-specific and/or tumor-associated antigens show characteristic expression patterns in various cancer types. For example, Her2/neu is expressed in 20-30% of breast cancers, ovarian cancers, gastric cancers, lung cancers, and such. NY-ESO-1 is expressed in 20-30% of breast cancers, ovarian cancers, and esophageal cancers; MAGE-A4 in 50-60% of head and neck cancers and esophageal cancers, and in 20% of lung cancers; and SAGE in 20% of lung cancers, esophageal cancers, and head and neck cancers. The tumor-specific or tumor-associated antigen employed in the invention is HER-2/neu. Furthermore, some of the cancer/testis antigens such as NY-ESO-1, have been known to comprise in a single molecule 1) an antigenic site to bind an MHC class I molecule, thereby stimulating CD8+ CTL, and 2) an antigenic site to bind an MHC class II molecule, thus allowing identification as a SEREX antigen.

The tumor-specific or tumor-associated antigens activating CD8+ CTL cells exerts its function upon binding to an MHC class I molecule of a host animal to which a vaccine of the present invention is administered. When the host animal is a human, the antigen that activates CD8+ CTL cells needs to bind to a class I molecule of the HLA-A, -B or -C locus of each person. Incidentally, there are 23 known types of HLA-A locus, which is thought to be important in antigen presentation. Types A24 and A2 are common in Mongoloids including Japanese, and account for 70% or more. In Caucasians, including North American Caucasians and such, A1, A2, A3 and A29 are major types, and in Africans, A30 and A2 are common. Within its molecule, Her2/neu has been known to comprise epitopes that bind to HLA-A2 and/or HLA-A24. Tissue-specific antigens such as gp100, MART-1, and tyrosinase, as well as cancer/testis antigens such as MAGE-3 (A3), MAGE-A4, and NY-ESO-1, have also been known to comprise epitopes that bind to A2. Furthermore, tyrosinase and cancer/testis antigens such as MAGE-A4, NY-ESO-1, and SAGE, have been known to comprise an epitope that binds to A24. Accordingly, when a vaccine of the present invention is given to a host animal, it is preferable to examine the expression of these antigens in tumors, as well as the MHC type of the individual host animal, to select an antigen compatible to that type.

More specifically, the Genbank accession numbers for each antigen are shown below:
NY-ESO-1: AJ003149
BAGE1: AF499647
GAGE1:NM_001468
GAGE2: NM_001472
GAGE8: NM_012196
SAGE: NM_018666
HER-2/neu: M11730, NM_004448, NP_004439
MAGE-A3: NM_005362
MAGE-A4: NM_002362
MAGE-A12: NM_005367
DnaJ-like 2: NM_001539, NP_001530
PRAME:NM_006115
Homo sapiens hexamethylene-bis-acetamide-induced protein: XM_008348
Human retinoic acid-responsive protein: U50383
DIPA: XM_006503
FLJ20644fis: AK000651

In the present invention, the polynucleotides that encode the antigens are not restricted, and may be DNAs, RNAs, or such, as long as they can elicit a desired immune response when administered to a host animal by a method of the present invention. The polynucleotides that encode the antigens of the present invention may be those whose nucleotide sequence is artificially modified by one or more amino acid deletions, substitutions, insertions and/or additions, by known methods such as site-directed mutagenesis (see Current Protocols in Molecular Biology, John Wiley & Sons, Ausubel et al., Section 8.1-8.5 (1987)), as long as the polypeptide encoded by the polynucleotide can generate a desired immune response in the host. Furthermore, as long as they can elicit a desired immune response in the host, the polynucleotides may encode naturally occurring polypeptides with mutations. Such mutants existing in nature may be isolated by known hybridization techniques (see Current Protocols in Molecular Biology, John Wiley & Sons, Ausubel et al., Section 6.3-6.4 (1987)) and gene amplification techniques (PCR) (see Current Protocols in Molecular Biology, John Wiley & Sons, Ausubel et al., Section 6.1-6.4 (1987)).

Further, if a gene encoding an antigenic protein is known, one skilled in the art can readily analyze the hydrophobic and/or hydrophilic regions within the amino acid sequence of the protein (Kyte and Doolittle, J. Mol. Biol. 157: 105-122 (1982)) and its secondary structure (Chou and Fasman, Ann. Rev. Biochem. 47: 251-276 (1978)) to predict antigenic regions in the amino acid sequence (for example, see Anal. Biochem. 151:540-546 (1985)), and can synthesize peptides having the predicted amino acid sequence to determine their antigenicity using the PEPSCAN method (Nature 314 (1985); Published Japanese Translation of International Publication No. Sho 60-500684), etc. Thus, a polynucleotide encoding a peptide fragment that comprises an epitope site that was determined based on an above-described method may be produced by techniques such as chemical synthesis, and used in an expression vector for antigens of the present invention.

Expression vectors used in the present invention are recombinant vectors into which an antigen gene of the present invention, and preferably an interferon gamma gene, are inserted. The vectors to insert these genes include plasmids, phages, cosmids, viruses, and other conventional vectors in the technical field of the present invention. Those skilled in the art can construct various plasmids and vectors based on techniques described in, for example, Molecular Cloning: A Laboratory Manual, Cold Spring Harbor Laboratory, N.Y (1989), edited by Sambrook *et al.,* and the above-described literature, edited by Ausubel *et al.*

Those skilled in the art can appropriately select factors such as promoters and terminators - used herein to regulate expression in the host - from known regulatory sequences, depending upon the host type and the purpose, and can also arrange them upstream and/or downstream of the antigen gene. Therefore, regulatory sequences derived from an antigen, or heterogeneous regulatory sequences, may be used in the present invention. Furthermore, if needed, markers such as antibiotic resistance markers may be used in the expression vectors of the present invention. Though many commercially available vectors can be used, nonessential polynucleotide sequences are preferably deleted from the vector in the present invention. The same holds for expression vectors for the interferon gamma gene. In addition, the polynucleotides encoding an antigen recognized by CD4+ helper T cells, those encoding a tumor-specific or tumor-associated antigen, and those encoding interferon gamma used in the present invention may be included in different expression vectors, or constructed to be expressed from a single vector, as long as they are arranged such that they can be expressed in the same cell.

Upon introduction into animal tissues, a vaccine of the present invention induces *in vivo* expression of a present antigen and elicits a desired immune response. Various methods are known for introducing nucleic acids into living bodies (T. Friedman, Science 244: 1275-1281 (1989)). Any method may be used as long as it induces *in vivo* expression of the present invention's antigens, and elicits a desired immune response.

The compositions of the present invention are useful as vaccines and can be used as naked plasmids. They may be packaged into liposomes, formed as various virus vectors including retrovirus vectors, adenovirus vectors, vaccinia virus vectors, poxvirus vectors, adeno-associated virus vectors, and HVJ (hemagglutinating virus of Japan) vectors (see K. Adolph "Virus genomic methods", CRC Press, Florida (1996), for example,), or coated on beads (carriers) such as colloidal gold particles. Preferably, vectors expressing an antigen recognized by CD4+ helper T cells, a tumor-specific, tumor-associated, or cell-related antigen, and preferably interferon gamma are adhered to a carrier particle, such as a gold particle, for introduction into the body by gene gun or such; however, the present invention is not limited to this. Methods for coating carrier particles with polynucleotides are known (see, for example, WO93/17706). Finally, the polynucleotides can be prepared in solutions suitable for *in vivo* administration, such as physiological saline. To enhance immune reactions, the compositions of the present invention may be prepared as vaccines in combination with adjuvants such as known proteins or other carriers. Moreover, agents such as calcium ions, which help the uptake of plasmids in a cell, may be used in combination. In addition, pharmaceutically acceptable agents that make transfection easy may also be used as required.

The polynucleotide vaccines of the present invention may be administered by any method, so long as the method allows the vaccine to generate an immune response within a host animal. Preferably, the compositions of the present invention are administered at a dose sufficient to induce an immune response in the host animal, by methods including injections or infusions via appropriate parenteral routes such as intravenous, intraperitoneal, subcutaneous, intradermal, adipose tissues, mammary gland tissues, inhalation, or intramuscular; gas induced particle bombardment methods (with gene guns or such); or application methods through mucosal routes using nasal drops or such forms. Furthermore, a host animal may be immunized by administering the present composition into a blood cell, bone marrow-derived cell (APC, etc.), and such by using *ex vivo* methods such as liposome transfection, particle bombardment methods, and viral infection, and then reintroducing the cell into the animal. Of the above-mentioned administration methods, gene transformation methods using accelerated particles are described in U.S. Patent No. 4,945,050m, and devices based on modified methods thereof are commercially available (BioRad Laboratories). In particular, a preferred result can be obtained by transfecting dendritic cells *ex vivo,* and then returning the cells to the body.

The types of host animals of the present invention are not limited, as long as the animal's tumor immune response is enhanced by a composition of the present invention. Specific examples include mammals such as mice, rats, bovines, pigs, and primates like monkeys and humans, etc. Preferable host animals of the present invention include primates, and particularly humans.

The doses of the compositions of the present invention depend on the immunogenicity of the ingredients to the host. However, those skilled in the art can determine appropriate doses required for immunization by administering a given dose of the composition to test animals, and measuring the antibody titer using assay methods such as ELISA, detecting CTL response by chromium release assay or such, or observing immune response by detecting Th response using cytokine release measurement. Those skilled in the art will recognize that the immunogenicity of an ingredient in a composition also depends on the strength of regulatory sequences, such as transcription and translation promoters, used in expression vectors of the present invention. Furthermore, those skilled in the art can readily adjust the dose of a composition of the present invention based on the types of expression vector to be used.

Thus, the present invention provides clinically useful vaccines for developing tumor-specific immunity *in vivo.* More specifically, the invention provides polynucleotide vaccines comprising expression vectors encoding an antigen recognized by CD4+ helper T cells which is NY -ES01 and a tumor-specific or tumor-associated antigen which is HER-2/neu, and preferably an expression vector encoding interferon gamma, as well as methods of using the same.

### Brief Description of the Drawings

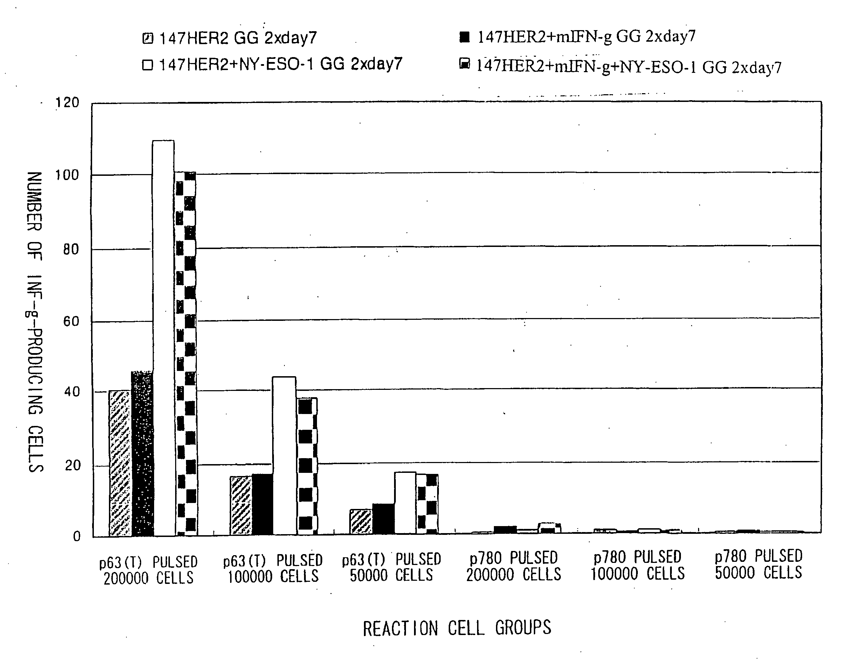

Fig. 1 is a graph showing the results of ELISPOT assays for the compositions of the present invention.

### Best Mode for Carrying Out the Invention

Herein below, the present invention will be specifically described using an example, however, it is not to be construed as being limited thereto.

### [Example 1]

The HER2-specific CD8 positive T cell enhancing effect of helper epitopes including NY-ESO-1

### [Method]

### 1) Plasmids

The following genes were incorporated into the expression vector pCAGGS to construct plasmids. Each of the purified plasmids were adjusted to 1 µg/µl, coated on to gold particles, and used for immunization using the Helios Gene Gun System.
- 147HER2: a gene encoding the 147 N-terminal amino acid residues of c-erbB-2/HER2/neu (HER2);
- p63(T) mini gene: a gene encoding the CTL epitope of HER2; namely, HER2p63(T), TYLPTNASL;
- NY-ESO-1: a gene encoding NY-ESO-1, which is a cancer/testis antigen
- mIFN-g: a gene encoding mouse interferon gamma.

### 2) Experimental Protocol

Six to eight week old BALB/c mice from the groups below (four animals per group) were immunized twice, at two weeks intervals. One week after immunization, two animals per group were sacrificed and CD8 positive T cells were prepared from their spleens using MACS system. Using P1.HTR (DBA/2 mice derived mastocytoma) pulsed with HER2p63(T) peptides as the target cell, ELISPOT assays were performed to detect interferon gamma producing cells. P1.HTR pulsed with the HER2p780 (PYVSRLLGI) peptide were used as a negative control. Each experiment was conducted in duplicate.

### (Experiment 1)

- 147HER2
- 147HER2 + mIFN-g
- 147HER2 + NY-ESO-1
- 147HER2 + mIFN-g + NY-ESO-1

### (Experiment 2)

- p63(T) mini gene
- p63(T) mini gene + mIFN-g
- p63(T) mini gene + NY-ESO-1
- p63(T) mini gene + mIFN-g + NY ESO-1

The results are shown in the following table and in Fig. 1.

| | Experiment 1 | Experiment 2 | Experiment 3 | Experiment 4 |
|---|---|---|---|---|
| p63(T) pulsed 200000 cells | 40.3 | 46 | 109.3 | 100.6 |
| p63(T) pulsed 100000 cells | 16.6 | 17.3 | 44 | 38 |
| p63(T) pulsed 50000 cells | 7 | 9 | 17.6 | 17 |
| p780 pulsed 200000 cells | 0.3 | 2 | 1 | 2.6 |
| p780 pulsed 100000 cells | 1 | 0.6 | 1 | 0.6 |
| p780 pulsed 50000 cells | 0.3 | 0.6 | 0.3 | 0.3 |

| | | | | |
|---|---|---|---|---|
| Ex. 1: 147HER2 GG 2x day 7 Ex.2: 147HER2 + mIFN-g GG 2x day 7 Ex.3: 147HER2 + NY-ESO- GG 2x day 7 Ex.4: 147HER2 + mIFN-g + NY ESCO-1 GG 2x day 7 ("GG" means administration using a gene gun.) | | | | |

### Industrial Applicability

As described above, it was clearly shown that a combination of an antigen recognized by CD4+ helper T cells and a tumor-specific or tumor-associated, antigen, and, more preferably, co-expression of interferon gamma with such a combination causes an increase in interferon gamma producing cells, and induces tumor-specific immunity. Examples of such antigen combinations include, for example, the combination of HER-2/neu and NY-ESO-1.

## Claims

1. A composition comprising one or more expression vectors encoding an antigen recognized by CD4+ helper T cells, and a tumor-specific or tumor-associated antigen, wherein the antigen recognized by CD4+ helper T cells is NY-ESO-1, and the tumor-specific or tumor-associated antigen is HER-2/neu.

2. The composition of claim 1, wherein polynucleotides encoding each antigen are comprised in separate expression vectors.

3. The composition of claim 2, wherein the expression vectors encoding each antigen have been immobilized on the same carrier particle.

4. The composition of claim 3, wherein the expression vectors encoding each antigen and an expression vector encoding interferon gamma have been immobilized on the same carrier particle.

5. A vaccine comprising the composition of any one of claims 1 to 4.

6. The vaccine of claim 5, wherein the vaccine is suitable for administration with a gene gun.

7. The vaccine of claim 5 or 6, wherein the vaccine is for prevention and/or treatment of cancers.

8. Use of the composition of any one of claims 1 to 4 in the manufacture of a medicament for inducing tumor-specific immunity in a mammal.

9. A composition of any one of claims 1 to 4 for use in inducing tumor specific immunity in an animal.

## Patentansprüche

1. Zusammensetzung, umfassend eine oder mehrere Expressionsvektoren, die für ein von CD4+ Helfer-T-Zellen erkanntes Antigen kodieren, und ein tumorspezifisches oder tumorassoziiertes Antigen, wobei es sich bei dem durch CD4+ Helfer-T-Zellen erkanntes Antigen um NY-ESO-1 und beim tumorspezifischen oder tumorassoziierten Antigen um HER-2/neu handelt.

2. Zusammensetzung nach Anspruch 1, wobei Polynucleotide, die für die einzelnen Antigene kodieren, in getrennten Expressionsvektoren enthalten sind.

3. Zusammensetzung nach Anspruch 2, wobei die für die einzelnen Antigene kodierenden Expressionvektoren am gleichen Trägerteilchen immobilisiert sind.

4. Zusammensetzung nach Anspruch 3, wobei die für die einzelnen Antigene kodierenden Expressionsvektoren und ein Expressionsvektor, der für Interferon-gamma kodiert, am gleichen Trägerteilchen immobilisiert sind.

5. Impfstoff, umfassend die Zusammensetzung nach einem der Ansprüche 1 bis 4.

6. Impfstoff nach Anspruch 5, wobei sich der Impfstoff zur Verabreichung mit einer Genpistole eignet.

7. Impfstoff nach Anspruch 5 oder 6, wobei der Impfstoff zur Verhinderung und/oder Behandlung von Krebs dient.

8. Verwendung der Zusammensetzung nach einem der Ansprüche 1 bis 4 bei der Herstellung eines Arzneimittels zur Induktion einer tumorspezifischen Immunität bei einem Säuger.

9. Zusammensetzung nach einem der Ansprüche 1 bis 4 zur Verwendung bei der Induktion einer tumorspzfischen Immunität bei einem Säuger.

## Revendications

1. Composition comprenant un ou plusieurs vecteurs d'expression codant pour un antigène reconnu par des lymphocytes T auxiliaires CD4+, et un antigène spécifique de tumeur ou associé à une tumeur, dans laquelle l'antigène reconnu par des lymphocytes T auxiliaires CD4+ est NY-ESO-1, et l'antigène spécifique de tumeur ou associé à une tumeur est HER-2/neu.

2. Composition de la revendication 1, dans laquelle les polynucléotides codant pour chaque antigène sont contenus dans des vecteurs d'expression séparés.

3. Composition de la revendication 2, dans laquelle les vecteurs d'expression codant pour chaque antigène ont été immobilisés sur la même particule porteuse.

4. Composition de la revendication 3, dans laquelle les vecteurs d'expression codant pour chaque antigène et un vecteur d'expression codant pour l'interféron gamma ont été immobilisés sur la même particule porteuse.

5. Vaccin comprenant la composition de l'une quelconque des revendications 1 à 4.

6. Vaccin de la revendication 5, dans lequel le vaccin est approprié pour une administration biolistique de gènes.

7. Vaccin des revendications 5 ou 6, dans lequel le vaccin sert à la prévention et/ou au traitement de cancers.

8. Utilisation de la composition de l'une quelconque des revendications 1 à 4 dans la préparation d'un médicament pour induire une immunité spécifique de tumeur chez un mammifère.

9. Composition de l'une quelconque des revendications 1 à 4 utilisable dans l'induction d'une immunité spécifique de tumeur chez un animal.
